(19) Europäisches Patentamt
European Patent Office
Office européen des brevets

(11) Veröffentlichungsnummer: **0 048 994**
**A1**

(12) # EUROPÄISCHE PATENTANMELDUNG

(21) Anmeldenummer: **81107764.3**

(22) Anmeldetag: **30.09.81**

(51) Int. Cl.³: **C 07 C 99/00**, C 07 C 101/16, C 07 C 153/09

(30) Priorität: **01.10.80 DE 3037159**

(43) Veröffentlichungstag der Anmeldung: **07.04.82 Patentblatt 82/14**

(84) Benannte Vertragsstaaten: **AT BE CH DE FR GB IT LI NL SE**

(71) Anmelder: **HOECHST AKTIENGESELLSCHAFT, Postfach 80 03 20, D-6230 Frankfurt/Main 80 (DE)**

(72) Erfinder: **Koch, Manfred, Dr., Kreuzheck 2, D-6239 Eppstein/Taunus (DE)**
Erfinder: **Hörlein, Gerhard, Dr., Assmannshäuser Weg 23, D-6000 Frankfurt am Main 71 (DE)**

(54) **Verfahren zur Herstellung von optisch aktiven 2-Anilino-propionsäureestern.**

(57) Herstellung von optisch aktiven 2-Anilinopropionsäureestern in hoher chemischer und optischer Ausbeute durch Umsetzung von optisch aktiven Milchsäureestersulfonaten mit einer zwei- bis zehnfachen Molmenge eines Anilins bei Temperaturen von 90 - 130°C in Abwesenheit von Lösungs- oder Verdünnungsmitteln.

EP 0 048 994 A1

0048994
HOECHST AKTIENGESELLSCHAFT HOE 80/F 224 Dr.GM/St

# Verfahren zur Herstellung von optisch aktiven 2-Anilinopropionsäureestern

Gegenstand der vorliegenden Erfindung ist ein Verfahren zur Herstellung von optisch aktiven 2-Anilinopropionsäureestern der allgemeinen Formel I,

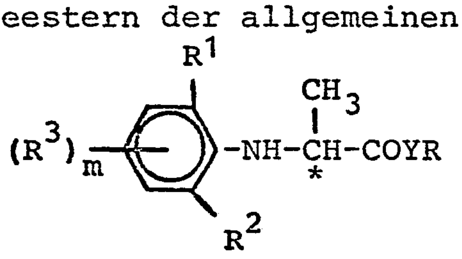
(I)

(*= Chiralitätszentrum)

worin R, $R^1$, $R^2$ und $R^3$ gleich oder verschieden sein können und jeweils einen $(C_1-C_4)$-Alkylrest bedeuten, Y für Sauerstoff oder Schwefel und m für die Zahlen 0 oder 1 stehen durch Umsetzung eines Milchsäureestersulfonates mit einem Anilin, dadurch gekennzeichnet, daß man optisch aktive Milchsäureestersulfonate der allgemeinen Formel II,

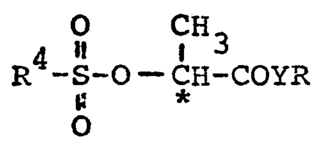
(II)

worin $R^4$ einen $(C_1-C_{12})$-Alkylrest oder einen gegebenenfalls durch $(C_1-C_2)$-Alkyl, Halogen oder die Nitrogruppe ein- oder zweifach substituierten Arylrest bedeutet und R, Y und * die Bedeutungen wie in Formel I haben, mit einer zwei- bis zehnfachen Molmenge eines Anilins der allgemeinen Formel III,

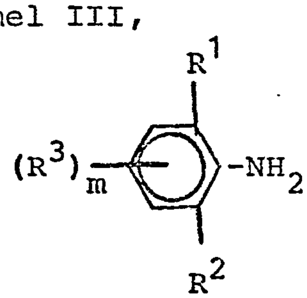
(III)

worin $R^1$, $R^2$, $R^3$ und m die Bedeutungen wie in Formel I haben, bei Temperaturen von 90 - 130°C, vorzugsweise bei 105 - 120°C, in Abwesenheit von Lösungs- oder Verdünnungsmitteln umsetzt.

Es ist aus J. Chem. Soc., Band 1961, S. 3303 - 3308 bekannt, daß man 2,6-Dimethylanilin und 2-Brompropionsäureäthylester in Benzol zu Äthyl-2-/(2,6-dimethylphenyl)-amino/-propionat umsetzen kann, jedoch wird bei einer Umsetzungszeit von 96 Stunden nur eine Ausbeute von 37 % der Theorie erreicht. Durch Anwendung eines dreifachen Überschusses an 2-Brompropionsäureester, bezogen auf 2,6-Dimethylanilin, und Zusatz von Natriumbikarbonat wird die Ausbeute auf 79,6 % verbessert (DE-OS 23 50 944, S. 15, Beispiel 1). Dagegen erhält man mit 2-Brompropionsäureäthylester eine nahezu quantitative Ausbeute, wenn die sterisch hindernden Substituenten in 2,6-Stellung fehlen, d.h., wenn z.B. Anilin als Ausgangsstoff umgesetzt wird. (Chem. Ber. 22 (1889) S. 1792 - 1795).

Bei der Umsetzung von Anilin mit 2-Chlorpropionsäuremethylester (12 Stunden, 125°C) unter Zusatz von Natriumacetat wird eine Ausbeute an dem gewünschten Anilinopropionester von 40 %, bei der gleichen Umsetzung mit o-Toluidin nur eine Ausbeute von 20 % der Theorie erzielt. (C.R. Acad. Sc. Paris Band 264, Serie C, 1967), S. 1064 - 65).

Eine weitere drastische Verringerung der Ausbeute auf 4,6 % resultiert bei der 36-stündigen Umsetzung des 2-Chlorpropionsäuremethylesters mit überschüssigem 2,6-Dimethylanilin in Xylol bei 135°C.

Bei der Herstellung optisch aktiver Verbindungen der Formel I ist neben einer guten chemischen Ausbeute der Erhalt einer hohen optischen Reinheit von besonderer Bedeutung und ein entscheidendes Kriterium.

Die üblicherweise angewandte Racemattrennung, ausgehend von racemischem Produkt unter Verwendung einer optisch aktiven Hilfssäure, ist nicht wirtschaftlich durchführbar. Die bei einem solchen Verfahren erforderliche fraktionierende Kristallisation ist sehr arbeitsaufwendig, zudem entstehen etwa gleiche Mengen des unerwünschten Enantiomeren.

Die direkte Synthese von optisch aktiven Verbindungen durch Umsetzung von reaktiven, optisch aktiven Propionsäureestern mit entsprechenden Anilinen ist nicht bekannt. Diese Art der Herstellung - eine nucleophile Substitution, welche vorwiegend unter Walden'scher Konfigurationsumkehr ablaufen soll - erscheint aus zweifacher Sicht schwierig.

Zum einen sind von den in Frage kommenden 2-Halogenpropionsäureestern nur die enantiomeren Formen der - wie oben gezeigt - reaktionsträgen 2-Chlorpropionsäureester, insbesondere der Methylester, in großtechnischem Maßstab verfügbar, während entsprechende 2-Brompropionsäureester aufgrund der zur Herstellung erforderlichen Spezialreaktionen nur in beschränktem Maße erhältlich bzw. herstellbar sind. Zum anderen tritt bei der Umsetzung von sowohl optisch aktiven 2-Chlor- wie 2-Brompropionsäureestern mit den entsprechenden Anilinen in erheblichem Umfang Racemisierung auf. So wird bei der 60-stündigen Umsetzung von L-2-Chlorpropionsäuremethylester mit überschüssigem 2,6-Dimethylanilin in Dimethylformamid bei 110°C racemisches Methyl-2-/N-(2,6-dimethylphenyl)-amino/-propionat erhalten (Ausbeute: 40 % d.Theorie).

Überraschenderweise wurde nun gefunden, daß es durch Umsetzung von optisch aktiven Milchsäureestersulfonaten der allgemeinen Formel II mit Anilinen der allgemeinen Formel III in Abwesenheit von Lösungs- oder Verdünnungsmitteln gelingt, die entsprechenden 2-Anilinopropionsäureester der allgemeinen Formel I in hoher chemischer und optischer Ausbeute herzustellen. Milchsäureestersulfonate sind z.T. bekannt (Can. J. Chem. 57 No. 2, S. 233 - 235 (1979)), ihre Herstellung erfolgt z.B. durch Umsetzung von D-, oder L-Milchsäureestern mit Sulfochloriden der Formel $R^4-SO_2Cl$, worin $R^4$ die Bedeutung wie in Formel I hat, in Gegenwart von Basen.

Bei dem erfindungsgemäßen Verfahren wird ein optisch aktives Milchsäureester-sulfonat der Formel II, bevorzugt das Mesylat ($R^4$ =$-CH_3$) oder das Tosylat ($R^4$ = $CH_3$-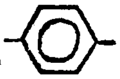-) des D- oder L-Milchsäuremethyl- oder äthylesters mit einem 2 bis 10-molaren, vorzugsweise 2,5 bis 6-molaren Überschuss eines Anilins der Formel III, bevorzugt 2,6-Dimethylanilin, bei Temperaturen von 90 - 130°C, bevorzugt 105 - 120°C, zur Reaktion gebracht, wobei pro Mol Verbindung der Formel II 1 Mol des Anilins *) zur Bindung der freiwerdenden Sulfonsäure unter Salzbildung benötigt wird. Die Reaktionszeiten sind nicht kritisch und betragen im allgemeinen zwischen 24 und 96 Stunden. Die optische Reinheit der erhaltenen Verbindungen der Formel I beträgt bei Einsatz von optisch reinen Milchsäureestersulfonaten der Formel II zwischen 75 und 90 % d. Theorie.

*) der Formel III

So wird z.B. bei der 48-stündigen Umsetzung von L-Milchsäuremethylestertosylat (R =$-CH_3$, $R^4$ = $CH_3$-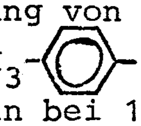-) mit der 2,5-fachen Molmenge 2,6-Dimethylanilin bei 115°C eine Ausbeute nach Destillation von 88 % d. Theorie und eine optische Reinheit von 86 % d. Theorie an D-Methyl-2-/N-(2,6-dimethylphenyl)-amino/-propionat erreicht.

Nach Beendigung der Umsetzung wird das gebildete Anilinhydrogensulfonat auf übliche Weise abgetrennt, entweder durch Auswaschen mit Wasser oder durch Filtration. Die vollständige Abtrennung durch Filtration kann durch Zusatz eines unpolaren Lösungsmittels, wie z.B. Toluol oder

Xylol erreicht werden. Die Reingewinnung von Verbindungen der Formel I erfolgt dann bevorzugt durch fraktionierte Destillation, bei der überschüssiges Anilin der Formel III als Vorlauf abdestilliert wird.

Die erfindungsgemäß erhältlichen Verbindungen der Formel I stellen wertvolle Zwischenprodukte dar, beispielsweise für die Herstellung von Fungiziden, wie z.B. in den DE-Patentanmeldungen P 29 30 145.9 und P 29 46 910.1 bereits vorgeschlagen.

Die Erfindung wird durch die nachstehenden Beispiele näher erläutert.

Beispiel 1

D-Methyl-2-[N-(2,6-dimethylphenyl)-amino]-propionat

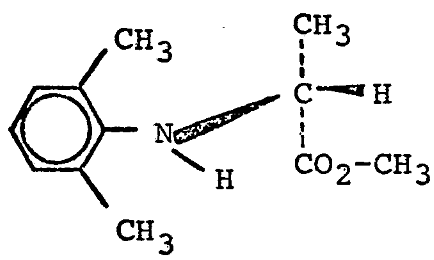

484 g (4 Mol) 2,6-Dimethylanilin und 258 g (1 Mol) L-Milchsäuremethylester-tosylat werden 54 Stunden bei 110°C gerührt. Nach Abkühlen werden dem Reaktionsgemisch 200 ml Toluol zugesetzt, 1 Stunde bei Raumtemperatur gerührt und 2,6-Dimethylanilinhydrogentosylat abfiltriert. Das Filtrat wird mit Wasser gewaschen, anschließend wird Toluol unter vermindertem Druck abdestilliert und das verbleibende Oel unter Vakuum rektifiziert. Man erhält 192 g (93 % d. Theorie) D-Methyl-2-[N-(2,6-dimethylphenyl)amino]propionat mit dem Siedepunkt 86°C / 0,025 mb und der spezifischen Drehung $[\alpha]_D^{25°C} = +18,52°$.

Beispiel 2

L-Äthyl-2-/N̄-(2,6-dimethylphenyl)-amino7-propionat

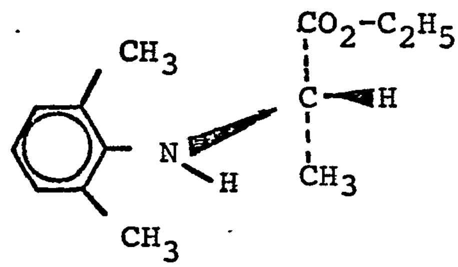

484 g (4 Mol) 2,6-Dimethylanilin werden mit 196 g (1 Mol) D-Milchsäureäthylester-mesylat 48 Stunden bei 110°C gerührt. Die Aufarbeitung des Reaktionsgemisches erfolgt wie im Beispiel 1 beschrieben. Man erhält 194 g (88 % d. Theorie) L-Äthyl-2-/N̄-(2,6-dimethylphenyl)-amino7-propionat mit dem Siedepunkt 87°C / 0,013 mb und einer spezifischen Drehung von $[\alpha]_D^{20°C} = -16{,}4°$.

Patentansprüche:

1. Verfahren zur Herstellung von optisch aktiven 2-Anilinopropionsäureestern der allgemeinen Formel I,

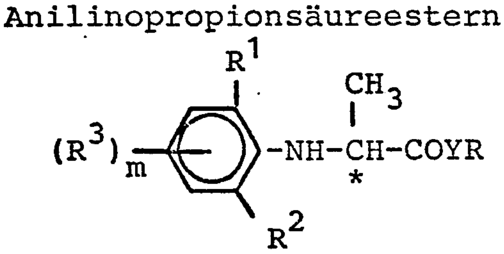

(I)

(* = Chiralitätszentrum)

worin R, $R^1$, $R^2$, $R^3$ gleich oder verschieden sein können und jeweils einen $(C_1-C_4)$-Alkylrest bedeuten, Y für Sauerstoff oder Schwefel und m für die Zahlen 0 oder 1 stehen, durch Umsetzung eines Milchsäureestersulfonates mit einem Anilin, dadurch gekennzeichnet, daß man optisch aktive Milchsäureestersulfonate der allgemeinen Formel II,

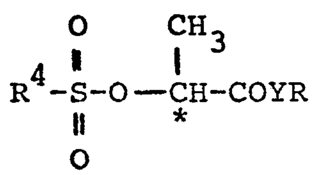

(II)

worin $R^4$ einen $(C_1-C_{12})$-Alkylrest oder einen gegebenenfalls durch $(C_1-C_2)$-Alkyl, Halogen oder die Nitrogruppe ein- oder zweifach substituierten Arylrest bedeutet und R, Y und * die Bedeutungen wie in Formel I haben, mit einer zwei- bis zehnfachen Molmenge eines Anilins der allgemeinen Formel III,

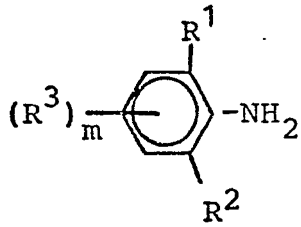

(III)

worin $R^1$, $R^2$, $R^3$ und m die Bedeutungen wie in Formel I haben, bei Temperaturen von 90 - 130°C in Abwesenheit von Lösungs- oder Verdünnungsmitteln umsetzt.

2. Verfahren nach Anspruch 1, dadurch gekennzeichnet, daß man die Umsetzung bei 105 bis 120°C durchführt.

3. Verfahren nach Anspruch 1 oder 2, dadurch gekennzeichnet, daß man einen 2,5 bis 6-molaren Überschuß des Anilins der Formel III einsetzt.

Europäisches Patentamt

# EUROPÄISCHER RECHERCHENBERICHT

0048994

Nummer der Anmeldung

EP 81 10 7764

## EINSCHLÄGIGE DOKUMENTE

| Kategorie | Kennzeichnung des Dokuments mit Angabe, soweit erforderlich, der maßgeblichen Teile | betrifft Anspruch | KLASSIFIKATION DER ANMELDUNG (Int. Cl.³) |
|---|---|---|---|
| X | DE - A - 2 650 434 (SHELL) * Ansprüche; Seite 7, Zeile 27 - Seite 8, Zeile 5; Beispiele * ---- | 1-3 | C 07 C 99/00 101/16 153/09 |

RECHERCHIERTE SACHGEBIETE (Int. Cl.³)

C 07 C 99/00
101/16
153/09

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 21-12-1981 | PAUWELS |

EPA form 1503.1 06.78